(19) Europäisches Patentamt
European Patent Office
Office européen des brevets

(11) Publication number: 0 262 684 A2

# (12) EUROPEAN PATENT APPLICATION

(21) Application number: 87114417.6

(22) Date of filing: 02.10.87

(51) Int. Cl.4: C07C 69/734 , C07C 67/31 , C07C 43/303 , C07C 41/50

(30) Priority: 03.10.86 US 914903
03.10.86 US 914904
03.10.86 US 914905

(43) Date of publication of application: 06.04.88 Bulletin 88/14

(84) Designated Contracting States: BE DE FR GB

(71) Applicant: NATIONAL DISTILLERS AND CHEMICAL CORPORATION
99 Park Avenue
New York, NY 10016(US)

(72) Inventor: Hanes, Ronnie M.
5817 Mt. Vernon Drive
Milford, Ohio(US)
Inventor: Baugh, William D.
405 Washington Avenue
Wilmington, Ohio(US)
Inventor: Chang, Biau-Hung
9743 Casey's Crossing
West Chester, Ohio(US)

(74) Representative: Patentanwälte Grünecker, Kinkeldey, Stockmair & Partner
Maximilianstrasse 58
D-8000 München 22(DE)

## (54) Conversion of an allylic ether to its corresponding acetal.

(57) The present invention is directed to a process for converting the ether group of an allylic ether to its corresponding acetal comprising contacting an allylic ether having acetal or carboxylate functionality with an organic hydroxy compound in the presence of a catalytically effective amount of a Group VIII metal compound at effective temperatures and pressures, said Group VIII metal being selected from the group consisting of cobalt and ruthenium.

EP 0 262 684 A2

## CONVERSION OF AN ALLYLIC ETHER TO ITS CORRESPONDING ACETAL

The present invention is directed to the conversion of an allylic ether to its corresponding acetal. More specifically, the present invention is directed to the conversion of an allylic ether having acetal or carboxylate functionality to its corresponding acetal by contacting the ether with an organic hydroxy compound in the presence of a catalytically effective amount of a Group VIII metal compound at effective temperatures and pressures, wherein said Group VIII metal is selected from the group consisting of cobalt and ruthenium.

The conversion of an allylic ether having acetal or carboxylate functionality to its corresponding acetal is of significant commercial potential. Such a step, for instance, can be utilized in a synthetic route to the formation of the commercially important compound azelaic acid from readily available butadiene. Other commercial applications have also been identified or proposed.

There is no known prior art disclosing a process for converting an allylic ether having acetal or ester (carboxylate) functionality to its corresponding acetal. The closest known prior art is Yamahara, et al., Japanese Patent Publication 53-37325. Yamahara, et al. disclose a process for converting an allylic ether to the corresponding acetal. This teaching emphasizes allylic ethers having no additional functionality. Yamahara, et al. make recitation of one species which possesses aldehyde (formyl) functionality. This teaching, however, is or limited commercial significance because of its emphasis on allylic ethers having no additional functionality. In the case where aldehyde functionality is present, the process suggested by the reference provides results which are not commercially exploitable.

The above remarks establish the need in the art for a new process for converting allylic ethers having acetal or carboxylate functionality to its corresponding acetal.

A new process has now been discovered which permits conversion of an allylic ether having acetal or carboxylate functionality to its corresponding acetal in commercially exploitable yield and selectivity.

In accordance with the present invention, a process for converting the ether group of an allylic ether to its corresponding acetal is provided. In this process, an allylic ether having acetal or carboxylate functionality is contacted with an organic hydroxy compound and a catalytically effective amount of a Group VIII metal compound wherein the Group VIII metal consists of cobalt and ruthenium. The corresponding acetal is formed from this process.

The present invention relates to a method for converting the ether group of an allylic ether having acetal or carboxylate functionality to the corresponding acetal. An allylic ether having acetal or carboxylate functionality has the formula

$R^1\text{-}CH=CH\text{-}CH_2\text{-}OR^2$    I

where $R^1$ is a cyclic or acyclic hydrocarbon group having from 1 to about 12, especially from 1 to about 10, carbon atoms substituted with an acetal or a carboxylate group. $R^1$ preferably is an alkyl, alkenyl, aryl or alkaryl group or an alkyl, alkenyl, aryl or alkaryl group having an ester, ether, formyl or ketone substituent in addition to the acetal or carboxylate group. The radical $R^2$ also is a cyclic or acyclic hydrocarbon group having from 1 to about 12 and especially from 1 to about 8 carbon atoms which is not substituted with an acetal or carboxylate group. Preferably, $R^2$ is an alkyl or aralkyl group. In the preferred embodiment wherein $R^1$ and $R^2$ are alkyl groups, structural isomers thereof are also intended to be included in the foregoing definition.

A preferred class of allylic ethers possessed of carboxylate functionality that are reacted to form an acetal in accordance with the present invention are alkyl n-alkoxy-(n-2)-alkenoate compounds. These compounds are characterized by the limitation that n is an integer equal to the number of carbon atoms in the main chain of the alkenoate group. It is emphasized that the alkenoate main chain may be substituted with 1 or more alkyl groups. The alkoxy groups is usually a lower alkoxy having from 1 to about 6 carbon atoms, preferably, from 1 to about 4 carbon atoms. The alkyl group usually contains 1 to about 6 carbon atoms, preferably, 1 to about 4 carbon atoms. Finally, the alkenoate group usually contains 3 to about 12 carbon atoms, preferably, 3 to about 10 carbon atoms.

A particularly preferred class of alkenoates within the class of alkyl n-alkoxy-(n-2)-alkenoates of the present invention is the case where the alkenoate main chain contains 9 carbon atoms, i.e., n is 9. That is, the case where the alkyl n-alkoxy-(n-2)-alkenoate is alkyl 9-alkoxy-7-nonenoate. These compounds having the structural formula

$$R^3O\text{-}\underset{\underset{O}{\|}}{C}\text{-}CH_2CH_2CH_2CH_2CH_2CH=CHCH_2\text{-}OR^2$$

II

where $R^3$ and $R^2$ in this formulation are the same or different and are $C_1$-$C_6$ alkyl. It is to be noted that the

radical $R^3O-\underset{\underset{O}{\|}}{C}-(CH_2)_5$

is included in the definition of $R^1$ in formula I hereinabove. More preferably, $R^3$ and $R^2$ are the same or different and are $C_1$-$C_4$ alkyl. Still more preferably, $R^3$ and $R^2$ are the same or different and are $C_1$-$C_2$ alkyl.

Among the compounds within the scope of the present invention are methyl 9-methoxy-7-nonenoate, methyl 9-ethoxy-7-nonenoate, methyl 9-propoxy-7-nonenoate, methyl 9-butoxy-7-nonenoate, methyl 9-pentoxy-7-nonenoate, methyl 9-hexoxy-7-nonenoate, ethyl 9-methoxy-7-nonenoate, ethyl 9-ethoxy-7-nonenoate, ethyl 9-propoxy-7-nonenoate, ethyl 9-butoxy-7-nonenoate, propyl 9-methoxy-7-nonenoate, propyl 9-ethoxy-7-nonenoate, propyl 9-propoxy-7-nonenoate, propyl 9-butoxy-7-noneonate, butyl 9-methoxy-7-nonenoate, butyl 9-ethoxy-7-nonenoate, butyl 9-propoxy-7-nonenoate, butyl 9-butoxy-7-nonenoate, pentyl 9-methoxy-7-nonenoate, pentyl 9-ethoxy-7-nonenoate, hexyl 9-methoxy-7-nonenonate, hexyl 9-ethoxy-7-nonenoate, and the like.

The compound of the formula II are formed by reacting an 8-alkoxy-1,6-octadiene with carbon monoxide and an alkanol in the presence of a cobalt catalyst to produce the alkyl 9-alkoxy-7-nonenoate. In a preferred embodiment, the cobalt catalyst is a cobalt coordination compound, preferably, cobalt carbonyl having the structural formula $Co_2(CO)_8$. The ligand, pyridine, is also preferably included in the cobalt coordination compound.

Another preferred class of allylic ethers that are converted to their corresponding acetals are those allylic ethers having acetal functionality and generically defined as 1,1,n-trialkoxy-(n-2)-alkene compounds. This preferred class of allylic ethers is characterized by an alkoxy usually having 1 to 6 carbon atoms, preferably, 1 to about 4 carbon atoms and an alkylene group usually having 3 to about 12 carbon atoms, preferably, 3 to about 10 carbon atoms. The alkene group can be substituted with one or more alkyl groups. The meaning of n in this preferred class of allylic ethers is an integer equal to the number of carbon atoms in the main chain of the alkene group.

A particularly preferred class of 1,1,n-trialkoxy-(n-2)-alkenes are the 1,1,9-trialkoxy-7-nonenes. That is, the case where the alkene chain contains 9 carbon atoms, i.e., where n is 9. Some examples of compounds within the broad class of the 1,1,9-trialkoxy-7-nonenes are 1,1,9-trimethoxy-7-nonene, 1,1,9-triethoxy-7-nonene, 1,1,9-tripropoxy-7-nonene, 1,1,9-tributoxy-7-nonene, and the like.

The process of the present invention involves reacting one of the aforesaid ethers with an organic hydroxy compound in the presence of a catalytically effective amount of a Group VIII metal compound, where the Group VIII metal is ruthenium or cobalt. The organic hydroxy compound comprises any saturated or unsaturated organic compound having at least one hydroxy group and is either a straight chain, branched chain, cyclic or heterocyclic compound and has from 1 to about 10 carbon atoms. Preferably, the organic hydroxy compound is an alkanol having from 1 to about 10 carbon atoms. More preferably, the alkanol is a lower alkanol having from 1 to about 6 carbon atoms. Still more preferably, the alkanol contains 1 to about 4 carbon atoms. Most preferably, the alkanol is methanol.

Other hydroxy compounds that may be used in accordance with the present invention include glycols such as 1,2-ethylene glycol, 1,2-propylene glycol, 1,3-propanediol glycol, 1,2-butanediol, 1,3-butanediol, and the like. Additionally, various mixtures of any of the foregoing organic hydroxy compounds may also be employed according to one embodiment of the present invention.

The Group VIII metal compound utilized in the present process is a ruthenium or cobalt-containing compound. The ruthenium compound of the present invention may be an organic ruthenium compound such as ruthenium carbonyl, and the like, or a ruthenium salt. Of these, ruthenium salts are preferred. Of the ruthenium salts, the ruthenium halides are more preferred. That is, the chlorides, bromides and iodides of ruthenium are particularly preferred. The most preferred halide is ruthenium chloride.

When the Group VIII metal is ruthenium, the reaction of the process of the present invention preferably occurs under anhydrous conditions at a temperature in the range of between about 120°C and about 200°C. More preferably, the temperature of reaction is in the range of between about 140°C and 170°C. The pressure of the reaction is autogenous pressure or that pressure that keeps the organic hydroxy compound from boiling off. Thus, the preferred pressure of the reaction is in the range of between about 200 and 400 psig.

The cobalt compound of the present invention is preferably a cobalt coordination compound or a cobalt salt.

In the preferred embodiment wherein a cobalt coordination compound is employed, the compound is preferably dicobalt octacarbonyl or a cobalt carbonyl with a ligand. The ligand may be a phosphine, a phosphite or a nitrogen-containing compound. Among the nitrogen-containing compounds preferred for use as the ligand are aliphatic amines such as triethylamine and heterocyclic aromatics such as pyridine. Of these, pyridine is particularly preferred.

In the alternate preferred embodiment wherein a cobalt salt is utilized, cobalt alkenoates and cobalt halides are particularly preferred. Of the halides, the chloride is usually employed.

In the case when the Group VIII metal is cobalt, it is preferred that carbon monoxide is utilized in the process of the present invention. Although carbon monoxide gas is not a reactant, its presence is important to the effectiveness of the cobalt catalyst in the catalytic reaction of this invention.

The reaction of the process of the present invention preferably occurs at a temperature in the range of between about 140°C and about 250°C and a pressure in the range of between about 300 and 4,000 psig. More preferably, process of the present invention occurs under anhydrous conditions at a temperature in the range of between about 150°C and about 180°C and a pressure in the range of between about 500 and about 1,000 psig. This pressure is provided in whole or in part by carbon monoxide. In a preferred embodiment, it is desirable to include hydrogen gas in the reaction. Although the partial pressure of the hydrogen gas is relatively minor, compared to that of carbon monoxide, its presence improves the effectiveness of the process of this invention.

The following examples are given to illustrate the present invention. In that these examples are provided solely for illustrative purposes, the invention should not be limited thereto.

## EXAMPLE 1

A 71 ml Paar [trademark] bomb was charged with 3 ml 1,1,9-trimethoxy-7-nonene; 3 ml methanol and 0.1 g cobalt chloride. The bomb was purged three times with carbon monoxide. Thereafter, the bomb was pressurized to 500 psig with carbon monoxide gas. A final pressure of 600 psig was obtained by further pressurization with hydrogen gas. The pressurized bomb was heated to 160°C and held at these thermodynamic conditions for 6 hours.

The product of this reaction was analyzed by gas chromatographic means. This analysis indicated the presence of 0.25 ml of 1,1,9,9-tetramethoxynonane. This represented a yield of 8%.

## EXAMPLE 2

A Parr [trademark] bomb was charged with the same ingredients and amounts as in Example 1. The bomb was then purged four times with hydrogen gas. The bomb was thereafter pressurized to 300 psig with hydrogen gas and heated to 160°C. The bomb was maintained at these conditions for 6 hours.

The product of this reaction was analyzed by gas chromatographic means. The product was formed to include 0.2 mol of 1,1,9,9-tetramethoxynonane representative of a yield of 7%.

## EXAMPLE 3

A glass bottle was charged with 30 g methyl 9-methoxy-7-nonenoate (MMNE); 5.3 g pyridine; 19.2 g methanol; and 3.5 g pentamethylbenzene, a gas chromatography standard, and the contents mixed. It is noted that the methyl 9-methoxy-7-nonenoate was 71.5% pure. Thus, the MMNE contained 21.45 g MMNE; 4.06 g methyl 8-methoxy-2-methyl-6-octenoate (MMMO); 0.14 g of the desired product, methyl 9,9-dimethoxynonenoate (MDNA); 0.62 g methyl 8,8-dimethoxy-2-methyloctanoate (MDMO); and 3.73 g of the impurities derived from carbomethoxylation of 8-methoxy-1,6-octadiene. The mixture was added to a nitrogen gas-purged 300 ml autoclave reactor along with 1.02 g cobalt carbonyl having the structural formula $Co_2(CO)_8$ which was separately charged from a viral.

The reactor was sealed, purged three times with carbon monoxide and pressurized to 40 psig with hydrogen gas. The total pressure was increased to 690 psig by the addition of 650 psig carbon monoxide gas. The reactor was heated to 170°C and carbon monoxide gas added to bring the pressure up to 1,000 psig. The reactor was maintained at these conditions for 4 hours with samples taken hourly.

Each of the hourly samples was analyzed by gas chromatography means. The results of this sampling is tabulated below in Table 1.

TABLE 1

| Time, hr | Overall Conversion of MMNE and MMMO (%) | Overall Selectivity to MDNA and MDMO (%) |
|---|---|---|
| 1 | 95.3 | 93.1 |
| 2 | 96.0 | 88.3 |
| 3 | 96.9 | 83.2 |
| 4 | 97.6 | 81.4 |

Footnotes

MDNA is methyl 9,9-dimethoxynonenoate
MDMO is methyl 8,8-dimethoxy-2-methyloctanoate
MMNE is methyl 9-methoxy-7-nonenoate
MMMO is methyl 8-methoxy-2-methyl-6-octenoate

EXAMPLE 4

The autoclave reactor of Example 3 was charged with the same components in the same amounts as in Example 3. The reactor was pressurized to 500 psig (480 psig CO and 20 psig $H_2$) and heated to 170°C. The reactor was maintained at these conditions for 3 hours with samples taken at 30 minutes, 1 hr, 2 hrs and 3 hrs.

The results of this run are tabulated in Table 2.

TABLE 2*

| Time, hr | Overall Conversion of MMNE and MMMO (%) | Overall Selectivity to MDNA and MDMO (%) |
|---|---|---|
| 0.5 | 90.2 | 95.9 |
| 1 | 93.9 | 93.9 |
| 2 | 94.6 | 91.9 |
| 3 | 95.4 | 90.2 |

* Same meanings as defined in footnotes of Table 1.

EXAMPLE 5

Example 4 was repeated except that the temperature of reaction was changed to 150°C, and the time of reaction was increased to 4 hours. Samples were taken at 0.5 hr, 1 hr, 2 hrs, 3 hrs and 4 hrs.

The results of this example are summarized below in Table 3.

TABLE 3*

| Time, hr | Overall Conversion of MMNE and MMMO (%) | Overall Selectivity to MDNA and MDMO (%) |
|---|---|---|
| 0.5 | 62.3 | 85.9 |
| 1 | 87.7 | 82.9 |
| 2 | 95.4 | 89.5 |
| 3 | 97.3 | 92.6 |
| 4 | 97.8 | 86.8 |

* Same meanings as defined in footnotes of Table 1.

EXAMPLE 6

Example 4 was repeated except for the temperature of reaction which was maintained at 160°C.

The results of this example are tabulated in Table 4 below.

TABLE 4*

| Time, hr | Overall Conversion of MMNE and MMMO (%) | Overall Selectivity to MDNA and MDMO (%) |
|---|---|---|
| 0.5 | 85.0 | 92.1 |
| 1 | 94.2 | 90.6 |
| 2 | 96.4 | 91.2 |
| 3 | 96.4 | 90.6 |

* Same meanings as defined in footnotes of Table 1.

EXAMPLE 7

Example 3 was repeated with only minor differences in purity of the starting methyl 9-methoxy-7-nonenoate (MMNE) which contained 24.10 g MMNE; 2.04 g MMMO; 0.21 g MDNA; and 0.63 MDMO. The example differed from Example 3 also in that the cobalt catalyst included 0.026 g $Co_2(CO)_8$, but no pyridine. This example was conducted for 3 hours. Samples were taken at 0.5 hr, 1 hr, 2 hrs and 3 hrs.

The example is summarized below in Table 5.

TABLE 5*

| Time, hr | Overall Conversion of MMNE and MMMO (%) | Overall Selectivity to MDNA and MDMO (%) |
|---|---|---|
| 0.5 | 95.4 | 97.4 |
| 1 | 97.2 | 99.0 |
| 2 | 97.4 | 99.0 |
| 3 | 98.3 | 99.0 |

* Same meanings as defined in footnotes of Table 1.

EXAMPLE 8

Example 7 was repeated except for that it was run in the absence of hydrogen. The cobalt catalyst was 0.10 g $Co_2(CO)_8$.

The results of this example are tabulated in Table 6 below.

TABLE 5*

| Time, hr | Overall Conversion of MMNE and MMMO (%) | Overall Selectivity to MDNA and MDMO (%) |
|---|---|---|
| 0.5 | 21.8 | 95.9 |
| 1 | 41.6 | 89.4 |
| 2 | 67.7 | 91.1 |
| 3 | 81.9 | 93.1 |

* Same meanings as defined in footnotes of Table 1.

EXAMPLE 9

A 500 ml polytetrafluoroethylene lined stirred reactor was charged with 45 methyl 9-methoxy-7-nonenoate (27.4 g, 213 mmol), 45 ml methanol, 40 ml methyl oleate, a solvent, 20 ml methyl benzoate, a gas chromatography internal standard, and 0.2 g ruthenium trichloride hydrate (1.4% water). The reactor was purged 4 times with nitrogen. Therefter, the reactor was pressurized, utilizing nitrogen gas, to 200 psig. The reactor was heated to 140°C and maintained at 140°C and 200 psig for 1 hour. The product of this reaction was analyzed by means of gas chromatography. It was determined that the desired product, methyl 9,9-dimethoxynonenoate, was obtained in a yield of 27.4 g (118 mmol). This represented a conversion of 65% with a selectivity to the desired product of 81%.

EXAMPLE 10

A second run was conducted in accordance with the procedure of Example 9. However, in Example 10 the temperature of reaction was 120°C instead of the 140°C temperature of Example 9. Also, the reaction was run for two hours instead of one hour.

The resultant production of methyl 9,9-dimethoxynonenoate was 26.8 g (115 mmol) as determined by gas chromatographic means. Including other by-products, the reaction involved a conversion of 86% of the starting compound, methyl 9-methoxy-7-nonenoate with a selectivity to the desired product, methyl 9,9-dimethoxynonenoate, of 63%.

EXAMPLE 11

A 71 ml Parr [trademark] bomb was charged with 5 ml methanol, 5 ml 1,1,9-trimethoxy-7-nonene and 0.0137 g ruthenium trichloride hydrate containing 1.4% water. The bomb was purged four times with nitrogen gas and then pressurized with nitrogen gas to 200 psig. The temperature was raised to 150°C and retained at this condition for 4 hours. The resultant products of this reaction were analyzed by gas chromatographic means and were found to include 3.7 ml of 1,1,9,9-tetramethoxynonane. This represents a yield of 64%.

COMPARATIVE EXAMPLE 1

The allylic ether having acetal functionality of Example 11, 1,1,9-trimethoxy-7-nonene, was replaced with the corresponding allylic ether having aldehyde functionality 9-methoxy-7-nonenal and, but for that change, was reacted in accordance with the procedure of Example 11. That is, 5 ml methanol; 5 ml 9-methoxy-7-nonenal; and 0.0137 g of $RuCl_3$ hydrate (1.4% water) were charged into a 71 ml Parr [trademark] bomb and reacted under the same thermodynamic conditions for the same period of time as Example 11. The resultant product, the same as in Example 11, 1,1,9,9-tetramethoxynonane was obtained in the yield of 0.7 ml. This represents a yield of 12%.

The above comparative example illustrates the improved results obtained when utilizing an allylic ether having acetal functionality compared to the same reaction of allylic ether having aldehyde (formyl group) functionality in a single step reaction. Obviously, Examples 9 and 10 illustrate the same unexpectedly improved results obtained when an allylic ether having carboxylate functionality, that is, a carbalkoxy group, is provided compared to the case where aldehyde functionality is provided.

EXAMPLE 12

Preparation of Methyl 9-methoxy-7-nonenoate

A 300 ml stirred reactor, after being purged with nitrogen, was charged with 65.0 g (0.464 mol) 8-methoxy-1,6-octadiene; 29.7 g (0.927 mol) methanol; and 33.1 g (0.418 mol) pyridine. These ligands had orginally been combined in a bottle prior to being charged into the reactor. Separately, 3.17 g (9.27 mmol) $Co_2(CO)_8$ was added to the stirred reactor. Upon sealing, the reactor was purged three times with carbon monoxide, pressurized to 40 psig with hydrogen and then to 3,500 psig with carbon monoxide. After stirring for a few minutes, the reactor was heated to 150°C and the pressure adjusted to 4,000 psig with carbon monoxide. The reactor was maintained at these conditions for three hours.

Upon reaction completion, the product was analyzed by gas chromatography and found to include methyl 9-methoxy-7-nonenoate in a yield of 46.3%.

Methyl 9-methoxy-7-nonenoate was isolated and characterized by spectral data. An infrared spectrum analysis was performed on the neat product with the following results (all measured in $cm^{-1}$); 3,020(m-w); 2,920(s); 2,845(s-m); 2,810(m); 1,730(s); 1,660(w); 1,445(m); 1,425(m); 1,350(m-w); 1,245(m); 1,185(s-m); 1,160(s-m); 1,110(s-m); and 960(m).

Note: s = strong, m = medium and w = weak.

Nuclear magnetic resonance analysis yielded the following results:

$^1$H-NMR Spectrum (in $CDCl_3$) (δ,ppm) = 1.32-1.44 (multiplet,4H); 1.62 (quintet,2H); 2.05 (quartet,2H); 2.30 (triplet,2H); 3.29 (singlet,3H); 3.65 (singlet,3H); 3.84 (doublet,2H); and 5.42-5.78 (multiplet,2H).

$^{13}$C-NMR Spectrum (in $CDCl_3$) (ppm) = 24.552; 28.411; 28.520 31.850; 33,670; 51.038; 57.286; 72.883; 126.174; 133.902; and 173.514.

Mass Spectrum (characteristic peaks in m/e) was as follows: 200(M+), 185, 168, 136, 94, 71.

The above preferred embodiments and examples are given to illustrate the scope and spirit of the present invention. These embodiments and examples will make apparent, to those skilled in the art, other embodiments and examples. These other embodiments and examples are within the contemplation of the present invention. Therefore, the present invention should be limited only by the appended claims.

## Claims

1. A process for converting the ether group of an allylic ether to its corresponding acetal comprising contacting an allylic ether having acetal or carboxylate functionality with an organic hydroxy compound in the presence of a catalytically effective amount of a Group VIII metal compound at effective temperatures and pressures, said Group VIII metal in the Group VIII metal compound being selected from the group consisting of cobalt and ruthenium.

2. The process in accordance with Claim 1 wherein said allylic ether is possessed of carboxylate functionality.

3. The process in accordance with Claims 1 or 2 wherein said allylic ether is alkyl n-alkoxy-(n-2)-alkenoate where n is an integer equal to the number of carbon atoms in the main chain of the alkenoate group and where the alkenoate main chain can be substituted by one or more alkyl groups.

4. The process in accordance with Claim 3 wherein said alkyl contains 1 to about 6 carbon atoms; said alkoxy contains 1 to about 6 carbon atoms; and said alkenoate contains 3 to about 12 carbon atoms.

5. The process in accordance with Claim 4 wherein said alkenoate contains 9 carbon atoms.

6. The process in accordance with Claim 1 wherein said allylic ether is possessed of acetal functionality.

7. The process in accordance with Claims 1 or 6 wherein said allylic ether is 1,1,n-trialkoxy-(n-2)-alkene where n is an integer equal to the number of carbon atoms in the alkene chain group and the alkene main chain can be substituted with one or more alkyl groups.

8. The process in accordance with Claim 7 wherein said alkoxy contains 1 to about 6 carbon atoms and said alkene contains 1 to about 12 carbon atoms.

9. The process in accordance with any of Claims 3-8 wherein n is 9 and the alkene is nonene.

10. The process in accordance with any of Claims 1-9 wherein said Group VIII metal compound is a ruthenium salt, ruthenium halide or ruthenium chloride.

11. The process in accordance with Claim 10 wherein said contact between said allylic ether and said organic hydroxy compound occurs under anhydrous conditions at a temperature in the range of between about 120°C and about 200°C at a pressure in the range of between about 200 and 400 psig.

12. The process in accordance with Claims 10-11 wherein said temperature is in the range of between about 140°C and 170°C.

13. The process in accordance with Claim 1 wherein said Group VIII metal compound is a cobalt salt, cobalt halide, cobalt chloride, cobalt carbonyl complex or $Co_2(CO)_8$.

14. The process in accordance with Claim 13 wherein said cobalt carbonyl complex is a ligand selected from the group consisting of a phosphine, a phosphate or aliphatic amine, and a heterocyclic nitrogen containing aromatic, or is $Co_2(CO)_8$ and pyridine.

15. The process in accordance with Claims 13-14 in which carbon monoxide is additionally present.

16. The process in accordance with Claims 13-16 in which hydrogen is additionally present.

17. The process in accordance with Claims 13-16 wherein the contact between said allylic ether and said organic hydroxy compound occurs at a temperature in the range of between about 140°C and about 250°C and at a pressure in the range of between about 300 and about 4,000 psig.

18. The process in accordance with Claim 18 wherein said reaction occurs under anhydrous conditions at a temperature in the range of between 150°C and about 180°C and a pressure in the range of between about 500 and about 1,000 psig.

10. The process in accordance with any of Claims 1-18 wherein the organic hydroxy compound is an alkanol containing from 1-6 carbon atoms.

20. The process in accordance with Claim 19 wherein the alkanol is methanol.

21. The process in accordance with any of Claims 1-20 wherein said allylic ether is selected from the group consisting of methyl 9-methoxy-7-nonenoate and 1,1,9-trimethoxy-7-nonene.

22. A compound having the structural formula:

$$R^3O-\underset{\underset{O}{\|}}{C}-C-C-C-C-C-C=C-C-OR^2$$

wherein $R^3$ and $R^2$ are the same or different and are $C_1$-$C_6$ alkyl.

23. A compound in accordance to Claim 22 wherein $R^3$ and $R^2$ are the same or different and are $C_1$-$C_4$ alkyl.

24. A compound in accordance with Claims 22-23 wherein $R^3$ and $R^2$ are the same or different and are $C_1$-$C_2$ alkyl.

25. A compound in accordance with Claims 22-24 wherein said compound is methyl 9-methoxy-7-nonenoate.